Europäisches Patentamt

European Patent Office

Office européen des brevets

(11) Veröffentlichungsnummer: **0 184 754 B1**

(12) **EUROPÄISCHE PATENTSCHRIFT**

(45) Veröffentlichungstag der Patentschrift: **18.03.92**

(21) Anmeldenummer: **85115284.3**

(22) Anmeldetag: **02.12.85**

(51) Int. Cl.⁵: **A61K 37/54**, A61K 9/32

(54) **Pankreasenzympräparate und Verfahren zu deren Herstellung.**

(30) Priorität: **12.12.84 DE 3445301**

(43) Veröffentlichungstag der Anmeldung:
**18.06.86 Patentblatt 86/25**

(45) Bekanntmachung des Hinweises auf die
Patenterteilung:
**18.03.92 Patentblatt 92/12**

(84) Benannte Vertragsstaaten:
**AT BE CH DE FR GB IT LI LU NL SE**

(56) Entgegenhaltungen:
**CH-A- 163 891**
**DE-B- 1 063 758**
**FR-A- 1 537 651**
**GB-A- 986 254**

(73) Patentinhaber: **HOECHST AKTIENGESELL-
SCHAFT**
**Postfach 80 03 20**
**W-6230 Frankfurt am Main 80(DE)**

(72) Erfinder: **Fülberth, Werner, Dr.**
**Theodor-Storm-Strasse 11**
**W-6233 Kelkheim (Taunus)(DE)**
Erfinder: **Freuer, Hans-Georg**
**Ingelheimer Strasse 31**
**W-6000 Frankfurt am Main 71(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

An Pankreasenzympräparate, die für die Therapie ungenügender Verdauungsleistungen Anwendung finden, werden folgende Anforderungen gestellt:

Sie sollen eine ausreichende Menge Pankreasenzym mit guter Qualität enthalten. Dies setzt eine hohe Enzymaktivität voraus.

Der therapeutische Wert dieser Präparate ist aber nur dann sichergestellt, wenn deren galenische Verarbeitung der hohen Enzymaktivität angepaßt ist. Eine wichtige Voraussetzung für eine wirksame Substitutionstherapie ist unter anderem die rasche und vollständige Freisetzung der Enzyme im Verdauungstrakt. Die Zubereitungen sollen unter den pH-Bedingungen des Duodenums die verdauungswirksamen Fermente sofort freisetzen.

So ergeben sich für Pankreasenzymzubereitungen als wesentliche Qualitätsparameter die Ausgangsaktivität der Enzyme, Magensaftresistenz zum Schutz gegen den inaktivierenden Einfluß der Magensäure, Zerfallszeit und Freisetzungsgeschwindigkeit der Enzyme, wobei der Lipase-Freisetzung für eine wirksame Pankreasenzymsubstitution eine besondere Bedeutung zukommt.

Eine weitere Forderung bezieht sich auf die Stabilität der Fermente während des gesamten Herstellungsprozesses und im Verlauf der Lagerung.

Bei der Herstellung von Pankreasenzympräparaten, vor allem wenn sie einen hohen Prozentsatz organischer Fermenttrockenpräparate enthalten, sind substanzspezifische und verfahrenstechnische Schwierigkeiten zu überwinden, die den Anforderungen an diese Präparategruppe entgegenstehen. Es ist daher auch schon beschrieben worden, daß bei einer Vielzahl von Handelspräparaten die benötigten Enzyme nicht in genügender Menge, vor allem aber nicht zum geeigneten Zeitpunkt freigesetzt werden (H. Möller, Pharm. Ztg. 125, 2254 - 2258 (1980)). Auch herstelungs- bzw. lagerungsbedingte Enzymverluste konnten bei der vergleichenden Prüfung ermittelt werden. Auffallend ist aber auch, daß bei vielen Präparaten, insbesondere bei denen mit hohem Fermentanteil, die Anforderungen des Arzneibuches für magensaftlöslich bzw. magensaftesistent überzogene Tabletten nicht erfüllt werden.

Wenn auch Pankreatinpräparate mit einer mittleren Dosis von 300 - 400 mg noch den biopharmazeutischen Ansprüchen entsprechend formuliert werden können, so werden die Probleme doch offenkundig wenn es gilt, Pankreatinmengen von 700 - 800 mg in eine patientengerechte Arzneiform zu bringen, denn besonders bei den hochdosierten Präparaten ist im Vergleich zu den niedriger dosierten die Freisetzungsgeschwindigkeit sehr niedrig. Daher gibt es Literaturhinweise nur zur Herstellung verpreßter, rasch zerfallender Pankreatinzubereitungen mit einer mittleren Dosis von 300 - 400 mg (vgl. z.B. Deutsche Offenlegungsschrift 2 035 739 bzw. entspr. Derwent Ret. 08513 T (1972))

Eine Lehre zur Herstellung rasch freisetzender, hochdosierter Einfachdosisarzneiformen ist bisher nicht beschrieben worden.

Da die Freisetzungsgeschwindigkeit der Enzyme und die Zerfallscharakteristik verpeßter Pankreasenzympräparate in unmittelbarem Zusammenhang stehen, ist bei den hochdosierten Präparaten mit einem Pankreatingehalt von 700 - 800 mg die Verwendung eines hochaktiven Tablettensprengmittels unerläßlich. Die Verwendung von Tablettensprengmitteln bei Pankreasenzympräparaten ist schon beschrieben worden (vgl. E. Graf et al. Pharm. Ind. 44, 317 - 321 (1982)).

Die Schwierigkeit bei der Herstellung hochdosierter Pankreatinpräparate besteht darin, den Pankreatinkern, der zur raschen Enzymfreisetzung einen hohen Anteil eines hochaktiven Tablettensprengmittels enthalten soll, mit einem Schutzüberzug auf Basis organischer Lösungsmittel zu versehen. Die Schutzüberzüge sind gegen den inaktivierenden Einfluß des Magensaftes und zur Verminderung stabilitätsmindernder Umwelteinflüsse erforderlich. Auch verhindern sie während des Schluckvorgangs einen unerwünschten Kontakt mit dem Pankreasenzym.

Sie werden nach dem Stand der Technik als Lacke auf Basis organischer Lösungsmittel aufgetragen. Als besonders enzymschonende Lösungsmittel haben sich Chloroform, Aceton und Isopropanol bewährt. Wasser, vor allem in Verbindung mit Wärme, ist wegen seiner enzyminaktivierenden Wirkung zu vermeiden (vgl. E. Graf et al., Pharm. Ind. 45, 295 - 299 1983)).

Während des Lackiervorgangs dringt Lösungsmittel in die Kerne ein. Bei Anwesenheit von Sprengmitteln quellen die Kerne auf und werden während des Lackierprozesses mechanisch zerrieben. Sprengmittelfreie Kerne, wie sie niedrig dosierte Präprate aufweisen, lassen sich zwar lackieren, aber das unvermeidliche, im Kern zurückbleibende Restlösungsmittel (auch in Spuren) verändert den Film im Laufe der Lagerung physikalisch.

Es stellt sich nun das Problem, das Eindringen von Lösungsmitteln in die Filmtablettenkerne während des Lackiervorgangs zu vermeiden.

Nicht zuletzt ist dies deshalb erforderlich, weil geringe, im Kern verbleibende Restlösungsmittelmengen durch Einwirken auf das Tablettensprengmittel auch nach Lagerung, vor allem bei höheren Temperaturen, einen Quellungsdruck erzeugen. Es kommt dann zur Bildung von Haarrissen und zur Veränderung der Porenstruktur im Schutzfilm.

Ein zusätzlicher stabilitätsmindernder Faktor ergibt sich aus der Wechselwirkung zwischen Pankreatin und der Filmdecke. Bei Filmbildnern mit Estergruppen kann es im Laufe der Lagerung durch die in Pankreatin enthaltene Lipase zu einer Esterspaltung und somit zu einer chemischen Veränderung des Films kommen, die eine Beeinträchtigung der Magensaftresistenz bewirkt.

Es wurde nun überraschenderweise gefunden, daß man das Eindringen von Lösungsmitteln in Pankreatin-Kerne und somit auch den direkten Kontakt zwischen Kern und dem aus einem Lösungsmittel aufgesprühten Schutzfilm vermeiden kann, indem man die Pankreatin-Kerne zunächst mit einer Isolierschicht auf wäßriger Basis versieht.

Die Erfindung betrifft daher ein Verfahren zur Herstellung eines Pankreasenzympräparates in gepreßter Form, das dadurch gekennzeichnet ist, daß man auf den wirkstoffhaltigen Kern eine Isolierschicht auf wäßriger Basis, die aus Andecksirup enthaltend Zucker und gegebenenfalls Gelatine, arabisches Gummi, Stärke, Polyvinylpyrrolidon, hochdisperses Siliciumdioxid, Natriumcarboxymethylcellulose, Natriumalginat, Polyvinylalkohol oder Calciumcarbonat und einem Abdeckpuder enthaltend ein Gemisch ausgewählt aus den Substanzen Talkum, Calciumcarbonat, Puderzucker, hochdisperses Siliciumdioxid, Titandioxid und arabischer Gummi, besteht, aufträgt und anschließend mit einer Lackschicht auf Basis organischer Lösungsmittel versieht, so wie nach diesem Verfahren erhältliche Präparate.

Es war nicht vorherzusehen, daß gerade eine mit Wasser aufgetragene Isolierschicht den Pankreatin-Kern wirksam schützen könnte, da bekannt ist, daß gerade Wasser, vor allem unter Einfluß von Wärme enzyminaktivierende Wirkung hat.

Die Isolierschicht wird bei dem erfindungsgemäßen Verfahren in zwei Phasen aufgetragen. In der ersten Phase werden die Kerne mit einem, vorzugsweise erwärmten, Andecksirup versetzt, bis diese zu kleben beginnen. Anschließend gibt man so lange Andeckpuder hinzu, bis die Kerne wieder frei rollen. Vor dem Auftragen der nächsten Andeckschicht müssen die angedeckten Kerne gut getrocknet werden. Dies kann mit einem Luftgebläse vorgenommen werden.

Zum wirksamen Schutz gegen eindringende Lösungsmittel und zur Isolierung des Kerns gegen die Abschluß-Filmschicht reichen im allgemeinen zwei bis fünf dieser Andeckschichten aus.

Bei der Abschluß-Filmschicht handelt es sich um eine Lackschicht auf Basis organischer Lösungsmittel. Sie kann magensaftlöslich oder magensaftresistent sein.

Nach dem erfindungsgemäßen Verfahren werden sowohl niedrig dosierte Pankreasenzympräparate, das sind solche mit einem Wirkstoffgehalt bis etwa 500 mg, erhalten als auch hochdosierte, wobei diese etwa 500 bis 1000 mg Pankreasenzyme enthalten.

Bei niedrig dosierten Pankreasenzympräparaten sind Tablettensprengmittel nicht zwingend erforderlich. Somit entfallen weitestgehend die bei Anwesenheit hochaktiver Tablettensprengmittel vorhandenen Herstellungsprobleme. Es hat sich jedoch gezeigt, daß durch die Trennung des Kerns vom Abschlußlack die lagerungsbedingte Veränderung des Abschlußlacks durch den Einfluß von Restlösungsmitteln im Kern unterbleit und der lagerungsbedingte Verlust der Magensaftresistenz vermieden werden kann.

Dagegen ist der Zusatz eines Tablettensprengmittels bei der Herstellung hochdosierter Präparate erforderlich.

Die hochdosierten Pankreatinkerne mit einem hohen Anteil eines aktiven Tablettensprengmittels werden durch Andeckschichten auf wäßriger Basis wirksam gegen das Eindringen von Lösungsmitteln während des abschließenden Lackiervorgangs geschützt ohne dabei deren Enzymfreisetzungscharakteristik zu verändern.

Die Kerne werden durch die Andeckschicht ausreichend mechanisch stabilisiert, so daß während des Lackierprozesses kein Rollverschleiß feststellbar ist.

Die erfindungsgemäßen Zubereitungen enthalten z.B. 100 bis 1000 mg, vorzugsweise 500 bis 1000 mg, insbesondere 700 bis 800 mg Pankreatin pro Dosierungseinheit. Im Kern können sie weiter für Pankreasenzympräparate übliche Wirkstoffe wie Dimethylpolysiloxan, Rindergalle, Dehydrocholsäure, Hemicellulase, Bromelain, pflanzliche Lipasekonzentrate und 2-Äthoxy-6,9-diamino-acridin-D-L-lactat (bzw. andere physiologisch verträgliche Salze davon) enthalten.

Als zerfalls- und freisetzungsbeschleunigende Tablettensprengmittel kommen in einer Konzentration von 1 bis 20 Gew.-% (vorzugsweise 5 bis 15 %) bezogen auf den Pankreatinanteil folgende Sprengmittel in Betracht: quervernetzte Natriumcarboxymethylcellulose, niedrig substituierte Hydroxypropylcellulose, Natriumcarboxymethylstärke, Formaldehyd-Casein, quervernetztes Polyvinylpyrrolidon, Calcium-Carboxymethylcellulose, Natriumamylopektinglykolat. Zur Unterstützung der zerfallbeschleunigenden Wirkung der vorgenannten Desintegrationshilfsmittel kann man beispielsweise Natriumchlorid zusetzen.

Als weitere Hilfs- und Trägerstoffe kommen beispielsweise mikrokristalline Cellulose, Polyethylenglykol,

EP 0 184 754 B1

gepulverte Fasercellulose, hochdisperses Siliciumdioxid, Dicalciumphosphat-Dihydrat, Aluminiumhydroxidgel in Betracht.

Zur Isolierung des Pankreatin enthaltenden Kerns werden sogenannte Isolier- oder Andeckschichten aufgetragen. Diese in 2 Phasen aufgetragenen Andeckschichten bestehen aus einem Andecksirup z.B. wäßriger Zuckersirup, 50 bis 65 %, mit geringen Zusätzen wie Gelatine, arabisches Gummi, Stärke, Polyvinylpyrrolidon, hochdisperses Siliciumdioxid, Natriumcarboxymethylcellulose, Natriumalginat, Polyvinylalkohol, Calciumcarbonat. Sie bestehen weiterhin aus einem Andeckpuder. Hierfür werden vorzugsweise Gemische, enthaltend Talkum, Calciumcarbonat, Puderzucker, hochdisperses Siliciumdioxid, Titandioxid, arabische Gummi, eingesetzt. Die Abschlußfilmschicht kann sowohl magensaftlöslich als auch magensaftbeständig sein.

Die magensaftlöslichen Lacke enthalten beispielsweise folgende Filmbildner: Hydroxypropylcellulose, Hydroxypropylmethylcellulose, Hydroxyethylcellulose, Copolymerisat mit kationischem Charakter auf Basis von Dimethylaminoethylmethacrylat und neutralen Methacrylsäureestern (Eudragit[(R)]-E).

Die magensaftbeständigen Schutzhüllen bestehen beispielsweise aus Celluloseacetatphthalat, Hydroxypropylmethylcellulosephthalat, Polyvinylacetatphthalat, Copolymerisate mit anionischem Charakter auf Basis von Methacrylsäure und Methacrylsäureestern (Eudragit[(R)] L oder S).

Für beide Lacksysteme kommen noch Hilfsstoffe wie Titandioxid, Eisenoxidpigmente, Talkum und Weichmacher wie Polyethylenglykole, Rizinusöl, Citronensäuretriethylester, Phthalsäureester, Glyzerinfettsäureester in Betracht. Bei beiden Lacksystemen werden übliche organische Lösungsmittel, gegebenenfalls im Gemisch mit Wasser verwendet.

Die nach dem erfindungsgemäßen Verfahren erhaltenen Präparate, auch die hochdosierten, sind mechanisch sehr stabil. Der mechanische Schutz zeigte sich auch nach Lagerung bei +40°C. Magensaftresistente Pankreatin-Filmtabletten, die erfindungsgemäß hergestellt wurden, waren auch nach 6 Monaten (+ 40°C) äußerlich noch unverändert. Es zeigten sich keine Haarrisse an der Lackoberfläche und die Prüfung auf Magensaftresistenz entsprach den Anforderungen der Europäischen Pharmakopoe.

Die Lipasefreisetzung aus einem Pankreasenzympräparat ist ein Prüfparameter für die pharmazeutische Verfügbarkeit von Pankreatin. Sie wurde wie nachfolgend beschrieben für hochdosierte erfindungsgemäße Präparate und bekannte Präparate bestimmt.

Die bekannten Präparate I, II, III und IV hatten folgende Zusammensetzungen:

```
I:    Pankreatin                     700 mg
      gereinigte, getrocknete
      Ochsengalle                     50 mg        Filmtabletten
      Hilfsstoffe                    345 mg


II:   Pankreatin                     700 mg
      Gesamtgallen-                   50 mg
      säuren (Fel suis)-                           Filmtabletten
      (berechnet als Cholsäure)
      Hilsstoffe                      60 mg


III:  Pankreatin                     800 mg
      Hilfsstoffe                     92 mg        Filmtabletten


IV:   Pankreatin                     700 mg
      Trockenxtrakt aus              120 mg
      Aspergillus oryzae                           Filmtabletten
      Hilfsstoffe                    650 mg
```

4

Von den erfindungsgemäß hergestellten Präparaten wurden diejenigen der Beispiele 1 und 3 untersucht.

Die Prüfung der Lipasefreisetzung wurde nach der von H. Möller (Pharm. Ztg. 125, 2254 -2258 (1980)) beschriebenen Methode II vorgenommen:

Paddle Apparatur nach USP XX

| Prüfflüssigkeit: | NaCl | 2,0 g |
|---|---|---|
| | $NaH_2PO_4 \cdot H_2O$ | 9,2 g |
| | Aqua dest. ad | 1000 ml |

Die Prüfflüssigkeit wurde je nach Bedarf, auf pH 3 bzw. pH 6 mit 6N HCl bzw. NaOH conc. eingestellt.

Prüfflüssigkeit pH 3          0,75 Std.

Prüfflüssigkeit pH 6          2,25 Std.

600 ml Prüfflüssigkeit, 37°C, 100 U/min.

Die Prüfungsergebnisse sind in der folgenden Tabelle zusammengefaßt.

Vergleichende Bewertung der Lipase-Freisetzung nach H. Möller als Prüfparameter für die pharmazeutische Verfügbarkeit von Pankreatin

| Präparat | I | II | III | IV | entspr. Bsp. 1 | entspr. Bsp. 3 |
|---|---|---|---|---|---|---|
| Nachgewiesene Lipaseaktivität nach FIP Einheiten | 24 364 | 26 599 | 30 006 | 26 806 | 6 568 | 36 171 |
| freigesetzte Lipase-Aktivität in % nach | | | | | | |
| 45 Minuten | 2 | 2 | 1 | 1,5 | 6 | 1,3 |
| 75 Minuten | 2 | 2 | 2 | 3,5 | 6 | 8,5 |
| 135 Minuten | 3 | 3 | 2 | 63,2 | 63 | 74,1 |
| 180 Minuten | 5 | 3 | 3 | 63,5 | 100 | 96,7 |

Wie aus den Ergebnissen zu ersehen ist, wird bei den zum Vergleich geprüften Präparaten das für den Verdauungsvorgang wichtige Enzym Lipase nicht in genügender Konzentration und vor allem nicht zum geeigneten Zeitpunkt freigesetzt. Die erfindungsgemäß hergestellten Zubereitungen geben in dem für den Verdauungsprozeß wesentlichen Zeitraum d.h. innerhalb von 2 Stunden nach der Magenpassage das Leitenzym Lipase vollständig bzw. nahezu vollständig frei.

Eine Voraussetzung zur Beschleunigung der Enzymfreisetzung, vor allem bei Präparaten mit einer hohen Pankreatindosis, ist die Verwendung von hochaktiven Tablettensprengmitteln, die wiederum nur ihren Zweck erfüllen können, wenn der Pankreatinkern vor der Abschlußlackierung erfindungsgemäß mit einer Schutzschicht auf wäßriger Basis isoliert wurde.

Die nachfolgenden Beispiele sollen die Erfindung erläutern:

Beispiel 1

Herstellung von 10 000 Dragees:

1. Zur Herstellung der Drageekerne werden

1. 2,10 kg Pankreatin
2. 0,50 kg Hemicellulase
3. 0,25 kg Rindergalle, getrocknet
4. 0,15 kg Natriumchlorid
gemischt und durch Komprimieren auf einer Tablettenpresse oder Walze und anschließendes Zerkleinern zu einem Granulat von etwa 1,5 mm Korngröße verarbeitet.
3,0 kg des so hergestellten Granulates (1. bis 4.) werden zu Kernen mit einem Endgewicht von 300 mg verpreßt.

2. Zur Herstellung des Andecksirups werden

| 10,00 kg | Zucker unter Erhitzen in |
| 2,50 kg | demineralisertem Wasser gelöst |
| 12,50 kg = | Zuckerlösung A |
| 0,50 kg | Gelatine werden unter Erwärmen in |
| 2,50 kg | demineralisiertem Wasser gelöst |
| 3,00 kg = | Gelatinelösung B |
| 12,50 kg | Zuckerlösung A |
| 3,00 kg | Gelatinelösung B und |
| 3,75 kg | Stärkesirup, der vorher auf einem Wasserbad verflüssigt wurde, |
| 19,25 kg | werden gemischt |

3. Zur Herstellung des Andeckpuders werden Talkum und Calciumcarbonat zu gleichen Teilen gemischt.

Die Kerne werden in einem Dragierkessel auf folgende Weise mit 3 Andeckschichten (Isolierschichten) versehen:
Zunächst werden die im Kessel bewegten Kerne mit dem erwärmten Andecksirup 2. versetzt bis diese zu kleben beginnen.
Anschließend gibt man so lange Andeckpuder 3. hinzu, bis die Kerne wieder frei rollen. Dieser Vorgang wird, nach Einlegen von Trocknungsphasen, 3 mal wiederholt.
Die getrockneten Kerne werden auf die übliche Weise mit einem magensaftresistenten Schutzlack überzogen und anschließend dragiert.

Beispiel 2

Herstellung von 10 000 Filmtabletten
1. 5,00 kg Pankreatin
2. 0,35 kg mikrokristalline Zellulose
3. 0,40 kg Natriumchlorid
werden gemischt und durch Kompaktieren auf einer Tablettenpresse oder Walze und anschließendes Zerkleinern zu einem Granulat von etwa 1,5 mm Korngröße verarbeitet.
5,75 kg des so hergestellten Granulates werden in einem geeigneten Mischer mit
4. 0,50 kg quervernetztem Polyvinylpyrrolidon und
5. 0,05 kg hochdispersem Siliciumdioxid gemischt.
6,30 kg des Granulates (1. bis 5.) werden zu Tabletten mit einem Endgewicht von 630 mg verpeßt und auf die in Beispiel 1 beschriebene Weise mit 3 Andeckschichten versehen und anschließend, je nach Bedarf, mit einem magensaftlöslichen oder magensaftresistenten Schutzlack versehen.

Beispiel 3

Herstellung von 10 000 Filmtabletten
1. 8,00 kg Pankreatin und

6

EP 0 184 754 B1

2. 0,65 kg Natriumchlorid werden gemischt
8,65 kg dieser Mischung (1. - 2.) werden kompaktiert und durch anschließendes Zerkleinern zu einem Granulat von etwa 1,5 mm Korngröße verarbeitet.
8,65 kg Granulat (1. - 2.) werden mit
3. 0,55 kg mikrokristalliner Zellulose und
4. 0,80 kg quervernetztem Polyvinylpyrrolidon gemischt.
10,00 kg des Granulates (1. - 4.) werden zu Kernen mit einem Endgewicht von 1000 mg verpreßt und auf die in Beispiel 1 beschriebene Weise mit 3 Andeckschichten versehen. Nach dem Trocknen wird je nach Bedarf, ein magensaftlöslicher oder magensaftresistenter Schutzlack aufgetragen.

Beispiel 4

Herstellung von 10 000 Filmtabletten
1. 2,10 kg Pankreatin und
2. 0,49 kg mikrokristalline Zellulose werden gemischt und durch Kompaktieren und anschließndes Zerkleinern zu einem Granulat von etwa 1,5 mm Korngröße verarbeitet (Granulat A).
3. 0,80 kg Dimethylpolysiloxan und
4. 0,80 kg Hochdisperses Siliciumdioxid, hydrophob, werden gemischt.
1,60 kg dieser Mischung (3. - 4.) und
5. 0,80 kg mikrokristalline Zellulose werden gemischt.
2,40 kg der Mischung (3. - 5.) werden kompaktiert und durch Zerkleinern zu einem Granulat von etwa 1,5 mm Korngröße verarbeitet (Granulat B).
2,59 kg Granulat A (1. - 2.)
2,40 kg Granulat B (3. - 5.)
6. 0,08 kg Mikrokristalline Zellulose
7. 0,30 kg Quervernetztes Polyvinylpyrrolidon
8. 0,10 kg Hochdisperses Siliciumdioxid und
9. 0,03 kg Magnesiumstearat werden gemischt.
5,5 kg dieser Granulatmischung werden zu Kernen mit einem Endgewicht von 550 mg verpreßt und auf die in Beispiel 1 beschriebene Weise mit 3 Andeckschichten versehen.
Die angedeckten Kerne werden auf die übliche Weise mit einem magensaftresistenten Abschlußlack überzogen.

Beispiel 5

Herstellung von 10 000 Filmtabletten
1. 4,20 kg granuliertes Pankreatin
2. 0,50 kg getrocknete Rindergalle
3. 1,00 kg Hemicellulase
4. 0,30 kg Natriumchlorid
5. 0,30 kg Quervernetzte Natriumcarboxymethylcellulose
6. 0,17 kg Talkum
7. 0,03 kg Hochdisperses Siliciumdioxid werden gemischt und zu Kernen mit einem Endgewicht von 650 mg verpreßt. Die Kerne werden auf die in Beispiel 1 beschriebene Weise mit 3 Andeckschichten versehen und anschließend mit einem magensaftresistenten Schutzlack überzogen.

Beispiel 6

Herstellung von 10 000 Filmtabletten
1. 7,00 kg Pankreatin
2. 0,05 kg hochdisperses Siliciumdioxid und
3. 0,60 kg Natriumchlorid werden gemischt.
7,65 kg dieser Mischung (1. - 3.) werden kompaktiert und durch anschließendes Zerkleinern zu einem Granulat von etwa 1,5 mm Korngröße verarbeitet.
7,65 kg Granulat (1. - 3) werden mit
4. 0,80 kg mikrokristalliner Zellulose
5. 0,05 kg hochdispersem Siliciumdioxid und
6. 1,00 kg quervernetzter Natriumcarboxymethylzellulose gemischt.

7

9,50 kg des Granulates (1. - 6.) werden zu Kernen mit einem Endgewicht von 950 mg verpreßt und auf die in Beispiel 1 beschriebene Weise mit 3 Andeckschichten versehen.

Nach dem Trocknen wird ein magensaftlöslicher Schutzlack auf organischer Lösungsmittelbasis aufgetragen.

**Patentansprüche**

**Patentansprüche für folgende Vertragsstaaten : BE, CH, DE, FR, GB, IT, LI, LU, NL, SE**

1. Verfahren zur Herstellung eines Pankreasenzympräparates in gepreßter Form, dadurch gekennzeichnet, daß man auf den wirkstoffhaltigen Kern eine Isolierschicht auf wäßriger Basis, die aus einem Andecksirup enthaltend Zucker und gegebenenfalls Gelatine, arabischen Gummi, Stärke, Polyvinylpyrolidon, hochdisperses Siliciumdioxid, Natriumcarboxymethylcellulose, Natriumalginat, Polyvinylalkohol oder Calciumcarbonat, und einem Andeckpuder enthaltend ein Gemisch ausgewählt aus den Substanzen Talkum, Calciumcarbonat, Puderzucker, hochdisperses Siliciumdioxid, Titandioxid und arabischer Gummi, besteht, aufträgt und diesen Kern anschließend mit einer Lackschicht auf Basis organischer Lösungsmittel versieht.

2. Verfahren gemäß Anspruch 1, dadurch gekennzeichnet, daß man als Andecksirup Zuckersirup, der noch Gelatine und Stärke enthalten kann, verwendet.

3. Verfahren gemäß Anspruch 1, dadurch gekennzeichnet, daß man als Andeckpuder ein Gemisch aus Talkum und Calciumcarbonat verwendet.

4. Pankreasenzympräparat erhältlich nach Verfahren gemäß Anspruch 1.

5. Pankreasenzympräparat erhältlich nach Verfahren gemäß Anspruch 1, dadurch gekennzeichnet, daß es im Kern 500 bis 1000 mg Pankreasenzyme und 1 bis 20 Gew.-% bezogen auf Pankreasenzyme eines üblichen Sprengmittels enthält.

6. Pankreasenzympräparat erhältlich nach Verfahren gemäß Anspruch 1, dadurch gekennzeichnet, daß es im Kern 700 bis 800 mg Pankreasenzyme und 1 bis 20 Gew.-% bezogen auf Pankreasenzyme eines üblichen Sprengmittels enthält.

7. Pankreasenzympräparat erhältlich nach Verfahren gemäß Anspruch 1, dadurch gekennzeichnet, daß es im Kern noch weitere für Pankreasenzympräparate übliche Wirkstoffe enthält.

**Patentansprüche für folgenden Vertragsstaat : AT**

1. Verfahren zur Herstellung eines Pankreasenzympräparates in gepreßter Form, dadurch gekennzeichnet, daß man auf den wirkstoffhaltigen Kern eine Isolierschicht auf wäßriger Basis, die aus einem Andecksirup, enthaltend Zucker und gegebenenfalls Gelatine, arabisches Gummi, Stärke, Polyvinylpyrolidon, hochdisperses Siliciumdioxid, Natriumcarboxymethylcellulose, Natriumalginat, Polyvinylalkohol oder Calciumcarbonat, und einem Andeckpuder, enthaltend ein Gemisch ausgewählt aus den Substanzen Talkum, Calciumcarbonat, Puderzucker, hochdisperses Siliciumdioxid, Titandioxid und arabisches Gummi, besteht, aufträgt und diesen Kern anschließend mit einer Lackschicht auf Basis organischer Lösungsmittel versieht.

2. Verfahren gemäß Anspruch 1, dadurch gekennzeichnet, daß man als Andecksirup Zuckersirup, der noch Gelatine und Stärke enthalten kann, verwendet.

3. Verfahren gemäß Anspruch 1, dadurch gekennzeichnet, daß man als Andeckpuder ein Gemisch aus Talkum und Calciumcarbonat verwendet.

4. Verfahren gemäß Anspruch 1, dadurch gekennzeichnet, daß man Kerne mit einem Gehalt von 500 bis 1000 mg Pankreasenzyme verwendet und 1 bis 20 Gew.-% bezogen auf Pankreasenzyme eines üblichen Sprengmittels verwendet.

5. Verfahren gemäß Anspruch 1, dadurch gekennzeichnet, daß man Zubereitungen mit einem Pankreasenzymgehalt von 700 bis 800 mg und 1 bis 20 Gew.-% bezogen auf Pankreasenzymgehalt eines üblichen Sprengmittels herstellt.

6. Verfahren gemäß Anspruch 1, dadurch gekennzeichnet, daß man eine Zubereitung die im Kern noch weitere für Pankreasenzympräparate übliche Wirkstoffe enthält, herstellt.

**Claims**
**Claims for the following Contracting States : BE, CH, DE, FR, GB, IT, LI, LU, NL, SE**

1. A process for the preparation of a pancreatic enzyme product in compressed form, which comprises application to the core, which contains the active compound, of a water-based insulating layer which is composed of a primary covering syrup containing sugar and optionally gelatin, gum arabic, starch, polyvinylpyrrolidone, highly disperse silica, sodium carboxymethylcellulose, sodium alginate, polyvinyl alcohol or calcium carbonate, and a primary covering powder containing a mixture selected from the substances talc, calcium carbonate, powdered sucrose, highly disperse silica, titanium dioxide and gum arabic, and then providing this core of a lacquer layer based on organic solvents.

2. The process as claimed in claim 1, wherein sugar syrup, which can also contain gelatin and starch, is used as the primary covering syrup.

3. The process as claimed in claim 1, wherein a mixture of talc and calcium carbonate is used as the primary covering powder.

4. A pancreatic enzyme product obtainable by the process as claimed in claim 1.

5. A pancreatic enzyme product obtainable by the process as claimed in claim 1, which contains in the core 500 to 1,000 mg of pancreatic enzymes and 1 to 20% by weight, relative to pancreatic enzymes, of a customary disintegrant.

6. A pancreatic enzyme product obtainable by the process as claimed in claim 1, which contains in the core 700 to 800 mg of pancreatic enzymes and 1 to 20% by weight, relative to pancreatic enzymes, of a customary disintegrant.

7. A pancreatic enzyme product obtainable by the process as claimed in claim 1, which also contains in the core other active compounds customary for pancreatic enzyme products.

**Claims for the following Contracting State : AT**

1. A process for the preparation of a pancreatic enzyme product in compressed form, which comprises application to the core, which contains the active compound, of a water-based insulating layer which is composed of a primary covering syrup containing sugar and optionally gelatin, gum arabic, starch, polyvinylpyrrolidone, highly disperse silica, sodium carboxymethylcellulose, sodium alginate, polyvinyl alcohol or calcium carbonate, and a primary covering powder containing a mixture selected from the substances talc, calcium carbonate, powdered sucrose, highly disperse silica, titanium dioxide and gum arabic and then provision of a lacquer layer based on organic solvents.

2. The process as claimed in claim 1, wherein sugar syrup, which can also contain gelatin and starch, is used as the primary covering syrup.

3. The process as claimed in claim 1, wherein a mixture of talc and calcium carbonate is used as the primary covering powder.

4. The process as claimed in claim 1, wherein the core used contains 500 to 1,000 mg of pancreatic enzymes and 1 to 20% by weight, relative to pancreatic enzymes, of a customary disintegrant.

5. The process as claimed in claim 1 for the preparation of formulations containing 700 to 800 mg of pancreatic enzymes and 1 to 20% by weight, relative to the pancreatic enzyme content, of a customary disintegrant.

**6.** The process as claimed in claim 1 for the preparation of a formulation which also contains in the core other active compounds customary for pancreatic enzyme products.

**Revendications**

**Revendications pour les Etats contractants suivants : BE, CH, DE, FR, GB, IT, LI, LU, NL, SE**

**1.** Procédé pour la préparation d'une composition d'enzymes pancréatiques sous forme comprimée, caractérisé en ce que l'on applique sur le noyau contenant la substance active une couche isolante à base aqueuse qui est formée à partir d'un sirop de pré-enrobage contenant un sucre et éventuellement de la gélatine, de la gomme arabique, de l'amidon, de la polyvinylpyrrolidone, de la silice fortement dispersée, de la carboxyméthylcellulose sodique, de l'alginate de sodium, du poly(alcool vinylique) ou du carbonate de calcium, et d'une poudre de pré-enrobage contenant un mélange choisi parmi le talc, le carbonate de calcium, le sucre en poudre, la silice fortement dispersée, le dioxyde de titane et la gomme arabique, et l'on munit ensuite ce noyau d'une couche de laque à base de solvants organiques.

**2.** Procédé selon la revendication 1, caractérisé en ce que l'on utilise, en tant que sirop de pré-enrobage, un sirop de sucre qui peut contenir en outre de la gélatine et de l'amidon.

**3.** Procédé selon la revendication 1, caractérisé en ce que l'on utilise, en tant que poudre de pré-enrobage, un mélange de talc et de carbonate de calcium.

**4.** Composition d'enzymes pancréatiques que l'on peut obtenir conformément au procédé selon la revendication 1.

**5.** Composition d'enzymes pancréatiques que l'on peut obtenir conformément au procédé selon la revendication 1, caractérisée en ce qu'elle contient dans le noyau de 500 à 1 000 mg d'enzymes pancréatiques et de 1 à 20 % en poids, par rapport aux enzymes pancréatiques, d'un désintégrant usuel.

**6.** Composition d'enzymes pancréatiques que l'on peut obtenir conformément au procédé selon la revendication 1, caractérisée en ce qu'elle contient dans le noyau de 700 à 800 mg d'enzymes pancréatiques et de 1 à 20 % en poids, par rapport aux enzymes pancréatiques, d'un désintégrant usuel.

**7.** Composition d'enzymes pancréatiques que l'on peut obtenir conformément au procédé selon la revendication 1, caractérisée en ce qu'elle contient dans le noyau encore d'autres substances actives usuelles pour des compositions d'enzymes pancréatiques.

**Revendications pour l'Etat contractant suivant : AT**

**1.** Procédé pour la préparation d'une composition d'enzymes pancréatiques sous forme comprimée, caractérisé en ce que l'on applique sur le noyau contenant la substance active une couche isolante à base aqueuse qui est formée à partir d'un sirop de pré-enrobage contenant un sucre et éventuellement de la gélatine, de la gomme arabique, de l'amidon, de la polyvinylpyrrolidone, de la silice fortement dispersée, de la carboxyméthylcellulose sodique, de l'alginate de sodium, du poly(alcool vinylique) ou du carbonate de calcium, et d'une poudre de pré-enrobage contenant un mélange choisi parmi le talc, le carbonate de calcium, le sucre en poudre, la silice fortement dispersée, le dioxyde de titane et la gomme arabique, et l'on munit ensuite ce noyau d'une couche de laque à base de solvants organiques.

**2.** Procédé selon la revendication 1, caractérisé en ce que l'on utilise, en tant que sirop de pré-enrobage, un sirop de sucre qui peut contenir en outre de la gélatine et de l'amidon.

**3.** Procédé selon la revendication 1, caractérisé en ce que l'on utilise, en tant que poudre de pré-enrobage, un mélange de talc et de carbonate de calcium.

**4.** Procédé selon la revendication 1, caractérisé en ce que l'on utilise des noyaux contenant de 500 à 1 000 mg d'enzymes pancréatiques et de 1 à 20 % en poids, par rapport aux enzymes pancréatiques, d'un désintégrant usuel.

5. Procédé selon la revendication 1, caractérisé en ce que l'on prépare des compositions ayant une teneur en enzymes pancréatiques allant de 700 à 800 mg et contenant de 1 à 20 % en poids, par rapport à la teneur en enzymes pancréatiques, d'un désintégrant usuel.

6. Procédé selon la revendication 1, caractérisé en ce que l'on prépare une composition qui contient dans le noyau encore d'autres substances actives usuelles pour des composisitions d'enzymes pancréatiques.